# EUROPÄISCHE PATENTSCHRIFT

(11) **EP 1 633 689 B1**
(45) Veröffentlichungstag und Bekanntmachung des Hinweises auf die Patenterteilung: **11.04.2007**
(21) Anmeldenummer: 04739141.2
(22) Anmeldetag: 06.05.2004
(51) Int. Cl.: C07C 5/25, C07C 11/08

(54) **VERFAHREN ZUR DOPPELBINDUNGSISOMERISIERUNG BEI OLEFINEN**
METHOD FOR THE DOUBLE-BOND ISOMERISATION OF OLEFINS
PROCEDE D'ISOMERISATION A DOUBLE LIAISON POUR DES OLEFINES

(30) Priorität: 14.05.2003 DE 10321523
(43) Veröffentlichungstag der Anmeldung: 15.03.2006
(73) Patentinhaber: BASF Aktiengesellschaft, 67056 Ludwigshafen (DE)
(72) Erfinder: SIGL, Marcus, 68167 Mannheim (DE); STEINBRENNER, Ulrich, 67435 Neustadt (DE)
(86) Internationale Anmeldenummer: PCT/EP2004/004818
(87) Internationale Veröffentlichungsnummer: WO 2004/102488

(56) Entgegenhaltungen:
- US-A- 3 527 834
- US-A- 4 499 325

## Beschreibung

Die vorliegende Erfindung betrifft ein Verfahren zur Herstellung eines C₄- bis C₁₂-Olefins (Olefin A) aus einem anderen C₄- bis C₁₂-Olefin (Olefin B), und wobei sich Olefin (A) und Olefin (B) hinsichtlich der Lage der Doppelbindung unterscheiden, wobei man eine gasförmige Mischung enthaltend Olefin (B) und 0,01 bis 10 Gew.-%, bezogen auf die Gesamtmenge an Kohlenwasserstoffverbindungen in dieser Mischung, einer Verbindung mit einem Dipolmoment von 0,5 bis 5 Debye (Verbindung P) mit einem basischen Katalysator, bei einer Temperatur von 200 bis 700°C in Kontakt bringt, wobei es sich bei dem Katalysator um Natriumaluminat, Kaliumaluniniat, Natriumoxid oder Kaliumoxid auf Gamma-Aluniniumoxid oder auf einer Mischung von Gamma-Aluminiumoxic und Siliziumoxid handelt.

C₄- bis C₁₂ Olefine, z.B. Butene, sind wichtige Ausgangsverbindungen zur Herstellung höherveredelter Verbindungen. Sie werden z.B. in Steamcrackern durch Spaltung von Naphtha hergestellt. Die im Steamcracker gebildete Kohlenwasserstoffmischung entspricht jedoch oft nicht dem Bedarf an den einzelnen Kohlenwasserstoffen. Dies gilt auch für die Doppelbindungsisomere des Butens. Große Mengen an 1-Buten werden z.B. benötigt um hieraus durch Metathese 3-Hexen herzustellen oder durch Hydroformylierung C₅-Aldehyde. Es werden deshalb Verfahren benötigt, mit denen die einzelnen Doppelbindungsisomere ineinander umgewandelt werden können.

Es ist allgemein bekannt, dass die Isomerisierung von 2-Butenen nach 1-Buten eine Gleichgewichtsreaktion ist. Cis-2-Buten, trans-2-Buten und 1-Buten liegen im Gleichgewicht nebeneinander vor. Die thermodynamischen Daten sind in D. Stull, "The Chemical Thermodynamics of Organic Compounds", J. Wiley, New York 1969 aufgeführt.

In diesem Text wird "Isobuten" nicht unter "Butene" subsumiert.

In WO 02/094433 wird ein Verfahren zur Herstellung von 1-Buten aus 2-Butenen beschrieben, bei dem als Katalysatoren Magnesiumoxid, Calciumoxid, Bariumoxid Lithiumoxid oder deren Mischungen eingesetzt werden. Es wird jedoch ausdrücklich empfohlen (vgl. S. 7), polare Verbindungen wie Wasser und Alkohol aus dem Einsatzstoff zu entfernen.

US 4,217,244 beschreibt ebenfalls ein Verfahren zur Herstellung von 1-Buten aus 2-Butenen an einem Magnesiumoxid-Katalysator. Auch hier wird empfohlen, den Einsatzstoff mittels Behandlung durch Molsieb von Feuchtigkeit zu befreien (vgl. S. 4, Zeilen 22ff).

US 4499325 offenbart die Isomerisierung von 2-Buten zu 1-Buten bei 206°C in Gegenwart eines basischen Katalysators, der Natriumhydroxid enthält und bei 900°C calziniert wurde.

T. Yamaguchi et. al. beschreiben in Catalysis Surveys from Japan, Vol. 5, No. 2, April 2002, Seiten 81 ff die Doppelbindungsisomerisierung bei Olefinen an Alkalimetalloxidkatalysatoren auf Aluminiumoxid- oder Zirkondioxidträgern. Zur Herstellung der Katalysatoren werden die Träger zunächst mit Lösungen von Nitraten oder Carbonaten der Alkalimetalle imprägniert. Anschließend werden die imprägnierten Träger auf Temperaturen oberhalb der Zersetzungstemperatur der Nitrate bzw. Carbonate erhitzt, wobei die Alkalimetalloxide gebildet werden. HU-B-204021 offenbart ein Verfahren zur Herstellung von 1-Buten aus 2-Butenen an einem Alkalimetalloxid auf einem Aluminiumoxidträger.

Eine Aufgabe der vorliegenden Erfindung war es, ein Verfahren bereitzustellen, mit dem man Doppelbindungsisomere von Olefinen mit hoher Selektivität ineinander umwandeln kann. Eine weitere Aufgabe bestand darin, das Verfahren so zu gestalten, dass sich die bei den hohen Temperaturen (200 - 500°C) bekanntermaßen kurzen Standzeiten der eingesetzten basischen Kontakte verlängern. Insbesondere bezieht sich die Erfindung auf ein Verfahren, mit dem der 1-Buten-Anteil auf Kosten des 2-Butene-Anteils in C₄-Kohlenwasserstoffströmen erhöht werden kann.

Demgemäß wurde die eingangs definierte Erfindung gefunden.

Von besonderer Bedeutung ist das erfindungsgemäße Verfahren, wenn als Olefin (B) cis-2-Buten, trans-2-Buten, 1-Buten oder Mischungen hiervon eingesetzt werden. Meistens liegen die Butene in Form einer Mischung mit anderen Kohlenwasserstoffen wie n-Butan, iso-Butan oder Isobuten vor. Der Begriff Olefin (B) ist in diesem Text also so zu verstehen, dass er sich nicht auf einzelne Verbindungen bezieht sondern auch auf Mischungen verschiedener Olefine, in denen auch das gewünschte Isomerisierungsprodukt (Olefin A) und sonstige Kohlenwasserstoffverbindungen vorhanden sein können. Die Mengen an Olefin (A), die in solchen Mischungen vorhanden sind, liegt jedoch unterhalb der Menge, die im thermodynamischen Gleichgewicht bei der jeweiligen Reaktionstemperatur vorhanden ist. Sinngemäß das gleiche gilt für den Begriff Olefin (A). Hierunter werden auch Mischungen verschiedener Olefine und Kohlenwasserstoffe, in denen auch noch das als Ausgangsprodukt dienende Olefin (B) vorhanden sein kann, verstanden. Dies ergibt sich schon allein daraus, dass die Doppelbindungsisomerisierung eine Gleichgewichtsreaktion ist.

Bevorzugt wird das erfindungsgemäße Verfahren so ausgeführt, dass es sich bei Olefin (A) um einen 1-Buten-haltigen C₄-Kohlenwasserstoffstrom (1-C₄⁼-Strom) handelt und man zu dessen Herstellung als Olefin (B) einen 1-Buten- und 2-Butene-haltigen C₄-Kohlenwasserstoffstrom (1- und 2-C₄⁼-Feedstrom), dessen Gehalt an 1-Buten geringer ist als der, der am thermodynamischen Gleichgewicht bei der jeweiligen Reaktionstemperatur vorhanden ist. Selbstverständlich kann das Verfahren auch dazu genutzt werden um umgekehrt 1-Buten-reiche C₄-Kohlenwasserstoffströme in solche mit hohen 2-Butengehalt umzuwandeln.

Bei dem 1- und 2-C₄⁼-Feedstrom handelt es sich um C₄-Schnitte, die im Allgemeinen einen Gehalt an Butenen von 30 bis 100, bevorzugt 40 bis 98, besonders bevorzugt 50 bis 95 Gew.-% aufweisen. Neben den Butenen können im 1- und 2-C₄⁼-Feedstrom noch bis zu 10, bevorzugt bis zu 5 Gew.-% mehrfach ungesättigte Verbindungen oder Alkine, vor allem solche mit 3- oder 4-Kohlenstoffatomen wie Butadiene, Butine, Vinylacetylen, Propin und Propadien enthalten sein. Weiterhin können noch 0,5 bis 60, bevorzugt 1 bis 50 Gew.-% C₄-Alkane und Isobuten enthalten sein. Weitere Kohlenwasserstoffe mit mehr als 5 Kohlenstoffatomen, insbesondere Pentane und Pentene sind ggf. in Mengen bis maximal 10 Gew.-% enthalten.

Insbesondere eignen sich sog. Raffinate (Raffinat I oder II) als 1- und 2-C₄⁼-Feedstrom.

Solche Raffinate 1 sind herstellbar, indem man
- Naphtha oder sonstige Kohlenwasserstoffverbindungen einem Steamcracking- oder FCC-Prozess unterwirft und aus dem dabei gebildeten Stoffstrom eine C₄-Kohlenwasserstofffraktion abzieht
- aus der C₄-Kohlenwasserstofffraktion einen im wesentlichen aus Isobuten, 1-Buten, 2-Butenen und Butanen bestehenden C₄-Kohlenwasserstoffstrom (Raffinat 1) herstellt, indem man mittels Selektivhydrierung die Butadiene und Butine zu Butenen oder Butanen hydriert oder die Butadiene und Butine durch Extraktivdestillation entfernt.
Weiterhin sind die Raffinate I erhältlich, indem man
- aus einem Butane enthaltenden Kohlenwasserstoffstrom durch Dehydrierung und nachfolgende Isolierung der C₄-Olefine eine C₄-Olefin-Mischung herstellt
- aus der C₄-Olefin-Mischung einen im wesentlichen aus Isobuten, 1-Buten, 2-Butenen und Butanen bestehenden C4-Kohlenwasserstoffstrom-(Raffinat 1) herstellt, indem man mittels Selektivhydrierung die Butadiene und Butine zu Butenen oder Butanen hydriert oder die Butadiene und Butine durch Extraktivdestillation entfernt.

Aus dem Raffinat I kann das Raffinat II hergestellt werden, indem man aus dem Raffinat I den wesentlichen Anteil des Iso-Butens durch bekannte chemische, physikalischchemische oder physikalische Methoden abtrennt.

Nach einer 3. Methode lässt sich Raffinat II erhalten, indem man aus Methanol durch Dehydrierung eine C₄-Olefin-Mischung herstellt (MTO-Verfahren) und diese ggf. durch Destillation, Partialhydrierung oder Extraktivdestillation von Butadienen oder Alkinen befreit.

Zur weiteren Reinigung kann das Raffinat II durch Behandlung mit Adsorbermaterialien von Katalysatorgiften befreit werden.

Bei der Verbindung (P) handelt es ich bevorzugt um eine Verbindung mit einem Dipolmoment von 0,5 bis 5, bevorzugt 0,75 bis 4, besonders bevorzugt 1 bis 3 Debey. Damit die Verbindung (P) unter Reaktionsbedingungen in der Gasphase vorliegen kann, liegt ihr Siedepunkt bei Atmosphärendruck im Allgemeinen unter 200°C. Verbindungen mit solchen Eigenschaften sind dem Fachmann bekannt. Es handelt sich dabei z.B. um Sauerstoff- oder Stickstoff-haltige Verbidnungen, bevorzugt C₁- bis C₁₂-Alkylamine, C₂-bis C₆-Alkylendiamine wie Ethylendiamin, cyclischen Amine bei denen 1 oder 2 Stickstoffatome zusammen mit 1 oder 2 Alkandiylgruppen 5-, 6- oder 7-gliedrige Ringe bilden wie Piperazin, Triethylendiamin, C₁- bis C₁₂-Alkylalkohole, Alkylenglycole, C₂- bis C₁₂-Dialkylether, cyclischen Ether, bei denen 1 oder 2 Sauerstoffatome zusammen mit 1 oder 2 Alkandiylgruppen 5-, 6- oder 7-gliedrige Ringe bilden, wie Tetrahydrofuran oder Dioxan, Wasser oder Ammoniak. Der Begriff der Verbindung (P) umfasst auch Mischungen von Verbindungen, die das definitionsgemäße Dipolmoment aufweisen.

Die gasförmige Mischung, die als Einsatzstoff für das erfindungsgemäße Verfahren dient, enthält 0,01 bis 10 Gew.%, bevorzugt 0,05 bis 5 Gew.% der Verbindung (P), bezogen auf die Gesamtmenge an Kohlenwasserstoffverbindungen in dieser Mischung.

Für das Verfahren geeignet sind basische Katalysatoren, speziell Katalysatoren, die basische Metalloxide enthalten. Bevorzugt sind Erdalkalioxide, Alkalioxide, Erdalkalialuminate oder Alkalialuminate. Besonders bevorzugt enthalten geeignete Katalysatoren die Elemente Natrium oder Kalium. Ganz besonders bevorzugte Katalysatoren sind Natriumaluminat, Kaliumaluminat, Natriumoxid oder Kaliumoxid auf gamma-Aluminiumoxid oder einer Mischung von gamma-Aluminiumoxid und Siliziumdioxid.

Derartige Katalysatoren sind beispielsweise in folgenden Druckschriften beschrieben:

EP 718036_A1 empfiehlt die Verwendung von Erdalkalimetalloxiden geträgert auf Aluminiumoxid. DE 3319171_A und DE 3319099_A offenbaren die Verwendung von Oxiden der Erdalkalimetalle, Borgruppenelemente und Lantaniden auf gemischten Aluminiumoxid/Siliziumdioxid-Trägern. Die Dotierung von Magnesium-haltigen Al₂O₃-Kontakten mit Alkali oder Zirkon ist Gegenstand von US 4889840_A und US 4229610_A. HU 204021_B nennt eine Vorschrift zur Herstellung eines Katalysators durch Tränkung von Aluminiumoxid mit einer Alkaliverbindung und anschließender Kalzination. In US 4229610_A wird ein Katalysator bestehend aus Aluminiumoxid, Natriumoxid und Siliciumdioxid beschrieben. Yamaguchi et. al. beschreiben in Catalysis Surveys from Japan, Vol. 5, No. 2, April 2002, Seiten 81 ff die Doppelbindungsisomerisierung bei Olefinen an Alkalimetalloxidkatalysatoren auf Aluminiumoxid- oder Zirkondioxidträgern. Zur Herstellung der Katalysatoren werden die Träger zunächst mit Lösungen von Nitraten oder Carbonaten der Alkalimetalle imprägniert. Anschließend werden die imprägnierten Träger auf Temperaturen oberhalb der Zersetzungstemperatur der Nitrate bzw. Carbonate erhitzt, wobei die Alkalimetalloxide gebildet werden.

Die Katalysatoren, die in dem erfindungsgemäßen Verfahren eingesetzt werden, werden im Allgemeinen hergestellt, indem man
a) einen gamma-Aluminiumoxid enthaltenden Träger mit einer Lösung eines Alkali- oder Erdalkalinitrats, -acetats, -oxalats, -oxids, -hydroxyds, -hydrogencarbonats oder -carbonats imprägniert (Schritt a) und
b) den gemäß Schritt (a) getränkten Träger trocknet und anschließend bei einer Temperatur von 450 bis 850°C calciniert.

Die gamma-Aluminiumoxid enthaltenden Träger sind kommerziell erhältlich und zeichnen sich durch eine Oberfläche von 100 bis 400 m²/g und ein Porenvolumen von 0,1 bis 1,2 ml/g aus (gemessen durch Quecksilberporosimetrie).

Die Lösung, mit der die Träger in Schritt (a) imprägniert werden, können auch Mischungen von den genannten Salzen umfassen.

Die Menge an Lösung der vorgenannten Salze wird so bemessen, dass, wenn man unterstellt, dass die Gesamtmenge der Salze, mit der die Träger imprägniert werden, in Schritt (b) in die entsprechenden Alkali- oder Erdalkalimetalloxide überführt werden, das Gewicht an Alkali- oder Erdalkalioxid, bezogen auf das Gesamtgewicht des Katalysators, 2 bis 20, bevorzugt 5 bis 15 Gew.-% beträgt.

Die Katalysatoren werden üblicherweise im Festbett-, Wirbelbett- oder Wanderbett verwendet. Im praktischen Betrieb hat es sich herausgestellt, dass die Menge des 2-C₄⁼-Stroms, die pro Zeiteinheit über den Katalysator geleitet wird, 0,1 bis 40 g (2-C₄⁼-Strom)/[g (Katalysator) h] beträgt.

Für die Isomerisierung ist ein kontinuierlich durchströmtes Festbett-Reaktorsystem bevorzugt. Geeignete Reaktoren sind Rohrreaktoren, Rohrbündelreaktoren, Hordenreaktoren, Wickelreaktoren oder Wendelreaktoren. Die Umsetzung der 2-Butene zu 1-Buten ist endotherm. Die Temperaturkontrolle kann wie üblich durchgeführt werden. Zudem kann die Reaktion auch in einem adiabaten Reaktionssystem ausgeführt werden.

Olefin (B) kann flüssig oder gasförmig vorliegen. Wird Olefin (B) flüssig eingesetzt, so muss es vor der Reaktion verdampft werden. Der für die Verdampfung verwendete Apparat unterliegt dabei keiner Beschränkung. Es eignen sich übliche Verdampfertypen wie Naturumlaufverdampfer oder Zwangsumlaufverdampfer. Das Aufheizen des gasförmigen Olefin (B)-Stroms auf Reaktionstemperatur erfolgt in den üblicherweise verwendeten Apparaten z.B. Plattenwärmeüberträger oder Rohrbündelwärmeüberträger.

Verbindung P wird dem Olefin (B) vor der Reaktion zugesetzt. Die Zudosierung kann sowohl flüssig wie gasförmig erfolgen. Es ist jedoch sicherzustellen, dass Verbindung P bis zum Eintritt in den Reaktionsraum gasförmig vorliegt und auf Reaktionstemperatur ist. Zweckmäßigerweise wird Verbindung P zusammen mit dem Olefin (B) verdampft und aufgeheizt.

Die Isomerisierung wird bei einer Temperatur durchgeführt, bei der eine Verschiebung der Doppelbindung gewährleistet ist, Crackprozesse, Skelettisomerisierungen, Dehydrierungen und Oligomerisierungen, hingegen weitestgehend vermieden werden. Die Reaktionstemperatur liegt deshalb im Allgemeinen bei 200 bis 700, bevorzugt 250 bis 600, besonders bevorzugt bei 300 bis 500°C. Der Druck wird so eingestellt, dass das Olefin (B) gasförmig vorliegt. Er beträgt im Allgemeinen 0,1 bis 40, bevorzugt 1 bis 30, besonders bevorzugt 3 bis 20 bar.

Üblicherweise wird aus dem Olefin (A) die Verbindung (P) entfernt. Dies geschieht mit üblichen Trennmethoden. In einer speziellen Ausführungsform kann die abgetrennte Verbindung (P) recycliert werden und erneut dem Olefin (B) vor dem Eintritt in die Reaktionszone zugesetzt werden.

Für den Fall, dass Verbindung (P) Wasser ist, kann die Abtrennung in der kondensierten Phase durch einen Phasenabscheider erfolgen. In geringeren Mengen kann Wasser durch Molsieb oder eine Destillation des Azeotrops vom Olefin (A) getrennt werden.

Ein nach dem erfindungsgemäßen Verfahren hergestellter 1-C₄⁼-Strom eignet sich insbesondere für die Herstellung von 3-Hexen durch Metathese. Hierzu wird der 1-C₄⁼-Strom bei einer Temperatur von 20 bis 350°C mit einem üblichen Metathesekatalysator in Kontakt gebracht. Solche Metathesekatalysatoren sind allgemein bekannt und z.B. in der EP-A-1134271 beschrieben. Dabei handelt es sich im allgemeinen um Verbindungen eines Metalls der Vlb, Vllb oder VIII-Nebengruppe des Periodensystems der Elemente.

Sofern der 1-C₄⁼-Strom Alkine oder mehrfach ungesättigte Verbindungen enthält, so empfiehlt es sich, den 1-C₄⁼-Strom von den Verbindungen zu befreien, indem man ihn in Gegenwart eines Palladiumenthaltenden Katalysators einer Selektivhydrierung unterzieht, bei der eine Umsetzung von 1-Buten zu 2-Butenen praktisch unterbleibt. Eine solche selektive Hydrierung unter Vermeidung der Isomerisierung kann erreicht werden, indem man den 1-C₄⁼-Strom bei 40 bis 60°C und einem Wasserstoffpartialdruck von 0,5 bis 10⁶ Pascal mit einem Katalysatorbett aus einem Palladium-Trägerkatalysator in Kontakt bringt. Diese Art der Hydrierung ist allgemein bekannt und beispielsweise in der Monographie Petrochemical Processes, Volume 1, Synthesis-Gas Derivates and Major Hydrocarbons, A. Chauvel, G. Lefebvre, L. Castex, Institut Francais du Petrol Publications, von 1989, Editions Technip, 27 Rue Ginoux, 75737 Paris, Cedex 15, auf Seiten 208 und 209 beschrieben.

Ein nach dem oben beschriebenen Verfahren hergestellter 1-Buten reicher C4-Strom kann weiterhin für eine Vielzahl von Reaktionen als Ausgangsstoff eingesetzt werden. Beispielhaft seinen genannt: Dimerisierung, Oligomerisierung, Epoxidation, Carbonylierung und Copolymerisation mit Ethylen.

Besonders bevorzugt lässt sich das erfindungsgemäße Verfahren als Verfahrensschritt (b) in das in der DE-A 10311139.5 beschriebene Verfahren integrieren. Dies betrifft ein Verfahren zur Herstellung eines 1-Buten-haltigen C₄-Kohlenwasserstoffstroms (1-C₄⁼-Strom) aus einem 1-Buten- und 2-Butene-haltigen C4-Kohlenwasserstoffstrom (1- und 2-C₄⁼-Feedstrom), dessen Gehalt an 1-Buten geringer ist als der des 1-C₄⁼-Stroms, indem man
a) in einer Destillationskolonne den 1- und 2-C₄⁼-Feedstrom und einen mittels des nachfolgenden Schrittes (b) hergestellten 1-Buten- und 2-Butene-haltigen C₄-Kohlenwasserstoffstrom (1- und 2-C₄⁼-Kreisstrom), dessen Gehalt an 1-Buten geringer ist als der des 1-C₄⁼-Stroms, einspeist und aus der Destillationskolonne den 1-C₄⁼-Strom und einen 2-Butene-haltigen C4-Kohlenwasserstoffstrom (2-C₄⁼-Strom), dessen Gehalt an 1-Buten geringer ist als der des 1- und 2-C₄⁼-Feedstroms und des 1- und 2-C₄⁼-Kreisstroms, abzieht (Schritt a) und
b) aus dem 2-C₄⁼-Strom den 1- und 2-C₄⁼-Kreisstrom herstellt, indem man den 2-C₄⁼-Strom in einer Reaktionszone mit einem lsomerisierungskatalysator in Kontakt bringt, der die Umsetzung von 2-Butenen zu 1-Buten katalysiert (Schritt b).

### Experimenteller Teil

### Versuch 1

2-Buten der Fa. Linde wurde mit Ammoniak versetzt und die Mischung bei 40°C verdampft. Das Volumenverhältnis Butene zu Ammoniak im Dampf betrug laut GC-Analytik 1 zu 0,012. Über eine Gasdosierung wurden 8 Normliter/h bei Atmosphärendruck in einen Vorheizer (250°C) und anschließend in den auf 400°C geheizten Reaktor geleitet. Bei dem Reaktor handelte es sich um einen Wendelreaktor (d = 6 mm, I = 10 cm) der mit 5 g Katalysator gefüllt war und sich in einem elektrisch beheizten Umluftofen befand. Als Katalysator diente ein mit Kaliumcarbonat getränktes und bei 850°C kalziniertes gamma-Aluminiumoxid mit einem Kaliumgehalt von 5,4 Gew.%. Der Reaktoraustrag wurde über ein GC mit FID geleitet. Dabei wurden die in Tab. 1 aufgeführten Zusammensetzungen erhalten (Angabe erfolgt in GC-Flächen-%). Die Selektivität bezüglich linearer Butene lag über den gesamten Beobachtungszeit bei > 98%.

**Tab. 1. Zusammensetzung des Reaktionsaustrags, Ammoniak als Verbindung (P).**

| Laufzeit [h] | 1-Buten | cis-2-Buten | trans-2-Buten |
|---|---|---|---|
| 0 (Feed) | 0,2 | 72,3 | 27,2 |
| 11 | 25,1 | 37,1 | 37,2 |
| 41 | 25,6 | 36,1 | 37,8 |
| 71 | 25,3 | 38,4 | 35,7 |
| 100 | 25,6 | 35,9 | 38,0 |
| 130 | 25,3 | 34,8 | 39,3 |

### Versuch 2

2-Buten der Fa. Linde (60 g/h) und Wasser (1,3 g/h) wurden bei 6 bar Druck und 200°C verdampft. Die Mischung wurde auf Reaktionstemperatur (400°C) vorgewärmt und durch einen auf 400°C beheizten Rohrreaktor (d =10 mm, l = 1 m, 30 g Katalysator) geleitet. Als Katalysator diente ein mit Kaliumcarbonat getränktes und bei 850°C kalziniertes gamma-Aluminiumoxid mit einem Kaliumgehalt von 5,4 Gew.%. Der Reaktoraustrag wurde über ein GC mit FID geleitet. Dabei wurden die in Tab. 2 aufgeführten Zusammensetzungen erhalten (Angabe erfolgt in GC-Flächen-%). Die Selektivität bezüglich linearer Butene lag über den gesamten Beobachtungszeit bei > 98%.

**Tab. 2. Zusammensetzung des Reaktionsaustrags, Wasser als Verbindung (P).**

| Laufzeit [h] | 1-Buten | cis-2-Buten | trans-2-Buten |
|---|---|---|---|
| 0 (Feed) | 0,2 | 72,3 | 27,2 |
| 9 | 25,9 | 39,7 | 33,6 |
| 39 | 26,0 | 39,9 | 33,2 |
| 75 | 25,7 | 39,9 | 33,5 |
| 101 | 26,0 | 39,4 | 33,8 |
| 138 | 25,9 | 38,9 | 34,7 |
| 190 | 26,3 | 41,6 | 31,3 |

### Vergleichsversuch

Der Versuch wurde analog Versuch 1 durchgeführt. 2-Buten der Fa. Linde wird bei 40°C verdampft und 8 Normliter/h bei 400°C über den Katalysator geleitet. Der Reaktoraustrag wurde über ein GC mit FID geleitet. Dabei wurden die in Tab. 3 aufgeführten Zusammensetzungen erhalten (Angabe erfolgt in GC-Flächen-%).

**Tab. 3. Zusammensetzung des Reaktionsaustrags, ohne Verbindung (P).**

| Laufzeit [h] | 1-Buten | cis-2-Buten | trans-2-Buten |
|---|---|---|---|
| 0 (Feed) | 0,2 | 72,3 | 27,2 |
| 11 | 25,9 | 31,8 | 41,8 |
| 40 | 26,0 | 31,8 | 41,7 |
| 74 | 26,4 | 33,4 | 39,8 |
| 99 | 25,7 | 41,7 | 32,2 |
| 130 | 21,0 | 50,4 | 28,1 |

## Patentansprüche

1. Verfahren zur Herstellung eines C₄- bis C₁₂-Olefins (Olefin A) aus einem anderen C₄- bis C₁₂-Olefin (Olefin B), wobei sich Olefin (A) und Olefin (B) hinsichtlich der Lage der Doppelbindung unterscheiden, wobei man eine gasförmige Mischung enthaltend Olefin (B) und 0,01 bis 10 Gew.%, bezogen auf die Gesamtmenge an Kohlenwasserstoffverbindungen in dieser Mischung, einer Verbindung mit einem Dipolmoment von 0,5 bis 5 Debye (Verbindung P) mit einem basischen Katalysator bei einer Temperatur von 200 bis 700°C in Kontakt bringt, wobei es sich bei dem Katalysator um Natriumaluminat, Kaliumaluminat, Natriumoxid oder Kaliumoxid auf gamma-Aluminiumoxid oder auf einer Mischung von gamma-Aluminiumoxid und Siliziumdioxid handelt.

2. Verfahren nach Anspruch 1, wobei es sich bei Olefin (A) um einen 1-Buten-haltigen C₄-Kohlenwasserstoffstrom (1-C₄⁼-Strom) handelt und man zu dessen Herstellung als Olefin (B) einen 1-Buten- und 2-Butene-haltigen C₄-Kohlenwasserstoffstrom (1- und 2-C₄⁼-Feedstrom) einsetzt, dessen Gehalt an 1-Buten geringer ist als der des 1-C₄⁼-Stroms.

3. Verfahren nach Anspruch 2, wobei man einen 1- und 2-C₄⁼-Feedstrom einsetzt, bei dem das Verhältnis 2-Butene zu 1-Buten 6 : 1 bis 0,1 : 1 beträgt.

4. Verfahren nach Anspruch 2 oder 3, wobei man einen 1- und 2-C₄⁼-Feedstrom einsetzt, der maximal 5 Gew.-% mehrfach ungesättigte Verbindungen oder Alkine enthält.

5. Verfahren nach einem der Ansprüche 2 bis 4, wobei man einen 1- und 2-C₄⁼-Feedstrom einsetzt, bei dem der Gehalt an Butenen 30 bis 100 Gew.-% beträgt.

6. Verfahren nach einem der vorstehenden Ansprüche, wobei es sich bei Verbindung (P) um eine Sauerstoff- oder Stickstoff-haltige Verbindung handelt.

7. Verfahren nach einem der vorstehenden Ansprüche, wobei es sich bei Verbindung (P) um eine Verbindung, ausgewählt aus der Gruppe der C₁- bis C₁₂-Alkylamine, C₂- bis C₆-Alkylendiamine, cyclischen Amine, bei denen 1 oder 2 Stickstoffatome zusammen mit 1 oder 2 Alkandiylgruppen 5-, 6- oder 7-gliedrige Ringe bilden, C₁- bis C₁₂-Alkylalkohole, Alkylenglycole, C₂- bis C₁₂-Dialkylether, cyclischen Ether, bei denen 1 oder 2 Sauerstoffatome zusammen mit 1 oder 2 Alkandiylgruppen 5-, 6- oder 7-gliedrige Ringe bilden, Wasser oder Ammoniak handelt.

8. Verfahren nach einem der vorstehenden Ansprüche, wobei man einen Katalysator einsetzt, bei dem das Gewicht an Alkali- oder Erdalkalioxid, bezogen auf das Gesamtgewicht des Katalysators, 2 bis 20 Gew.% beträgt.

9. Verfahren nach einem der vorstehenden Ansprüche, wobei man einen Katalysator einsetzt, der erhältlich ist, indem man
a) einen gamma-Aluminiumoxid enthaltenden Träger mit einer Lösung eines Alkali- oder Erdalkalinitrats, -acetats, -oxalats, -oxids, -hydroxyds, - hydrogencarbonats oder-carbonats imprägniert (Schritt a) und
b) den gemäß Schritt (a) getränkten Träger trocknet und anschließend bei einer Temperatur von 450 bis 850°C calciniert.

## Claims

1. A process for preparing a C₄- to C₁₂-olefin (olefin A) from another C₄- to C₁₂-olefin (olefin B), where olefin (A) and olefin (B) differ with regard to the position of the double bond, by contacting a gaseous mixture comprising olefin (B) and from 0.01 to 10% by weight, based on the total amount of hydrocarbon compounds in this mixture, of a compound having a dipole moment of from 0.5 to 5 debye (compound P) with a basic catalyst at a temperature of from 200 to 700°C, wherein the catalyst is sodium aluminate, potassium aluminate, sodium oxide or potassium oxide, on gamma-aluminum oxide or on a mixture of gamma-aluminum oxide and silicon dioxide.

2. The process according to claim 1, wherein the olefin (A) is a 1-butenic C₄ hydrocarbon stream (1-C₄⁼ stream) and the olefin (B) used to prepare it is a 1-butenic and 2-butenic C₄ hydrocarbon stream (1- and 2-C₄⁼ feed stream) whose content of 1-butene is smaller than that of the 1-C₄⁼ stream.

3. The process according to claim 2, wherein a 1- and 2-C₄⁼ feed stream is used in which the ratio of 2-butenes to 1-butene is from 6 : 1 to 0.1 : 1.

4. The process according to claim 2 or 3, wherein a 1- and 2-C₄⁼ feed stream is used which comprises a maximum of 5% by weight of polyunsaturated compounds or alkynes.

5. The process according to any of claims 2 to 4, wherein a 1- and 2-C₄⁼ feed stream is used in which the content of butenes is from 30 to 100% by weight.

6. The process according to any of the preceding claims, wherein compound (P) is an oxygen or nitrogen compound.

7. The process according to any of the preceding claims, wherein the compound (P) is a compound selected from the group of the C₁- to C₁₂-alkylamines, C₂- to C₆-alkylenediamines, cyclic amines in which 1 or 2 nitrogen atoms together with 1 or 2 alkanediyl groups form 5-, 6- or 7-membered rings, C₁- to C₁₂-alkyl alcohols, alkylene glycols, C₂- to C₁₂-dialkyl ethers, cyclic ethers in which 1 or 2 oxygen atoms together with 1 or 2 alkanediyl groups form 5-, 6- or 7-membered rings, water or ammonia.

8. The process according to any of the preceding claims, wherein a catalyst is used in which the weight of alkali metal or alkaline earth metal oxide, based on the total weight of the catalyst, is from 2 to 20% by weight.

9. The process according to any of the preceding claims, wherein a catalyst is used which is obtainable by
a) impregnating a support comprising gamma-aluminum oxide with a solution of an alkali metal or alkaline earth metal nitrate, acetate, oxalate, oxide, hydroxide, hydrogencarbonate or carbonate (step a) and
b) drying the support saturated in step (a) and subsequently calcining it at a temperature of from 450 to 850°C.

## Revendications

1. Procédé de préparation d'une oléfine en C₄ à C₁₂ (oléfine A) à partir d'une autre oléfine en C₄ à C₁₂ (oléfine B), dans lequel l'oléfine (A) et l'oléfine (B) se différencient en fonction de la position de la liaison double, dans lequel un mélange gazeux contenant l'oléfine (B) et 0,01 à 10 % en poids, par rapport à la quantité totale des composés hydrocarbures de ce mélange, d'un composé ayant un moment dipolaire de 0,5 à 5 Debye (composé P) est mis en contact avec un catalyseur basique à une température de 200 à 700°C, dans lequel il s'agit, en ce qui concerne le catalyseur, d'aluminate de sodium, d'aluminate de potassium, d'oxyde de sodium ou d'oxyde de potassium sur de l'oxyde d'aluminium gamma ou sur un mélange d'oxyde d'aluminium gamma et de dioxyde de silicium.

2. Procédé selon la revendication 1, dans lequel il s' agit, en ce qui concerne l'oléfine (A), d'un flux d'hydrocarbures en C₄ contenant du 1-butène (flux de 1-C₄⁼), pour la préparation duquel un flux d'hydrocarbures en C₄ contenant du 1-butène et du 2-butène (flux d' alimentation de 1-C₄⁼ et de 2-C₄⁼), dont la teneur en 1-butène est plus faible que celle du flux de 1-C₄⁼, est utilisé en tant qu'oléfine B.

3. Procédé selon la revendication 2, dans lequel un flux d'alimentation de 1-C₄⁼ et de 2-C₄⁼ est utilisé, le rapport du 2-butène sur le 1-butène étant de 6:1 à 0,1:1.

4. Procédé selon la revendication 2 ou 3, dans lequel un flux d' alimentation de 1-C₄⁼ et de 2-C₄⁼, contenant au maximum 5 % en poids de composés polyinsaturés ou d'alcynes, est utilisé.

5. Procédé selon l'une quelconque des revendications 2 à 4, dans lequel un flux d'alimentation de 1-C₄⁼ et de 2-C₄⁼ est utilisé, la teneur en butènes étant de 30 à 100 % en poids.

6. Procédé selon l'une quelconque des revendications précédentes, dans lequel il s'agit, en ce qui concerne le composé (P), d'un composé contenant de l'oxygène ou de l'azote.

7. Procédé selon l'une quelconque des revendications précédentes, dans lequel il s'agit, en ce qui concerne le composé (P), d'un composé choisi dans le groupe des alkylamines en C₁ à C₁₂, des alkylènediamines en C₂ à C₆, des amines cycliques, pour lesquelles 1 ou 2 atomes d'azote forment en association avec 1 ou 2 groupes alcanediyle des cycles pentagonaux, hexagonaux ou heptagonaux, des alkylalcools en C₁ à C₁₂, des alkylèneglycols, des dialkyléthers en C₂ à C₁₂, des éthers cycliques, pour lesquels 1 ou 2 atomes d'oxygène forment en association avec 1 ou 2 groupes alcanediyle des cycles pentagonaux, hexagonaux ou heptagonaux, eau ou ammoniac.

8. Procédé selon l'une quelconque des revendications précédentes, dans lequel un catalyseur, pour lequel le poids en oxyde alcalin ou alcalino-terreux par rapport au poids total du catalyseur est de 2 à 20 % en poids, est utilisé.

9. Procédé selon l'une quelconque des revendications précédentes, dans lequel un catalyseur pouvant être obtenu
a) en imprégnant une substance porteuse contenant de l'oxyde d'aluminium gamma avec une solution d'un nitrate, d'un acétate, d'un oxalate, d'un oxyde, d'un hydroxyde, d'un hydrogénocarbonate ou d'un carbonate, alcalin ou alcalino-terreux, (étape a) et
b) en séchant la substance porteuse imprégnée conformément à l'étape (a) et en la calcinant ensuite à une température de 450 à 850°C, est utilisé.
